Europäisches Patentamt

European Patent Office

Office européen des brevets

(11) Veröffentlichungsnummer : **0 114 783**
**B1**

(12) **EUROPÄISCHE PATENTSCHRIFT**

(45) Veröffentlichungstag der Patentschrift :
15.04.87

(51) Int. Cl.⁴ : **C 07 D253/06**, A 01 N 43/64

(21) Anmeldenummer : 84810009.5

(22) Anmeldetag : 06.01.84

(54) Herbizides Triazin-Derivat, Verfahren zu seiner Herstellung, Mittel, welche das Triazin-Derivat enthalten, die Verwendung des Triazin-Derivats oder es enthaltender Mittel sowie ein zur Herstellung des Triazin-Derivats entwickeltes Zwischenprodukt, seine Herstellung und seine Verwendung.

(30) Priorität : 12.01.83 CH 150/83

(43) Veröffentlichungstag der Anmeldung :
01.08.84 Patentblatt 84/31

(45) Bekanntmachung des Hinweises auf die Patenterteilung : 15.04.87 Patentblatt 87/16

(84) Benannte Vertragsstaaten :
AT BE CH DE FR IT LI NL SE

(56) Entgegenhaltungen :
DE-A- 2 165 554
FR-A- 1 547 854
FR-A- 2 482 421
US-A- 3 671 523
CHEMICAL ABSTRACTS, Band 92, Nr. 13, 31. März
1980, Seite 143, Nr. 105167n, Columbus, Ohio, US H.
PARLAR u.a.: "The significance of quantum yield
during determination of environmental photochemical
degradability of organic compounds"
Z. Naturforsch. C. Bionci, 75, 30C, p. 499-504
Z. Naturforsch, B. Anorg. Chem., Org. Chem, 76, 31B,
1122-6.

(73) Patentinhaber : CIBA-GEIGY AG
Klybeckstrasse 141
CH-4002 Basel (CH)

(72) Erfinder : BÖHNER, BEAT, DR.
HÜGELWEG 3
CH-4102 Binningen (CH)

**0 114 783**

**Beschreibung**

Die vorliegende Erfindung betrifft das Triazin-Derivat 3,4-Di-(methylamino)-6-tert.butyl-4,5-dihydro-1,2,4-triazin-5-on, Verfahren zu seiner Herstellung, Mittel, welche das Triazin-Derivat enthalten und die Verwendung des Triazin-Derivats oder es enthaltender Mittel zur Bekämpfung von unerwünschtem Pflanzenwuchs.

Substituierte 1,2,4-triazin-5-on-Derivate, ihre herbiziden Eigenschaften und ihre Herstellung sind aus der US-PS 3 671 523 bekannt. Das 3,4-Di-(methylamino)-6-tert.butyl-4,5-dihydro-1,2,4-triazin-5-on ist in der vorgenannten Publikation nicht genannt.

Es wurde nun gefunden, dass das neue Triazin-Derivat 3,4-Di-(methylamino)-6-tert.butyl-4,5-dihydro-1,2,4-triazin-5-on ausgezeichnet zur Bekämpfung von unerwünschtem Pflanzenwuchs, insbesondere zur Bekämpfung von Unkräutern in Kulturpflanzenbeständen, geeignet ist.

3,4-Di-(methylamino)-6-tert.butyl-4,5-dihydro-1,2,4-triazin-5-on lässt sich z. B. herstellen, indem man

a) 3-Methylthio-4-methylamino-6-tert.butyl-4,5-dihydro-1,2,4-triazin-5-on mit Methylamin umsetzt, oder

b) 3-Methylamino-4-amino-6-tert.butyl-4,5-dihydro-1,2,4-triazin-5-on mit einem Methylhalogenid oder Dimethylsulfat umsetzt.

Das in der Verfahrensvariante b) verwendete 3-Methylamino-4-amino-6-tert.butyl-4,5-dihydro-1,2,4-triazin-5-on und seine Herstellung sind in der US-PS 3 671 523 beschrieben.

Die Umsetzung gemäss Verfahrensvariante a) wird zweckmässigerweise in Gegenwart eines Lösungs- oder Verdünnungsmittels durchgeführt. Beispiele dafür sind aliphatische und aromatische Kohlenwasserstoffe, wie beispielsweise Benzol, Toluol, Xylole, Petroläther, Cyclohexan, n-Hexan; halogenierte Kohlenwasserstoffe wie Chlorbenzol, Methylenchlorid, Aethylenchlorid, Chloroform, Tetrachlorkohlenstoff, Tetrachloräthylen; Alkohole, insbesondere aliphatische Alkohole, wie beispielsweise Methanol, Aethanol, Propanole wie beispielsweise 2-Propanol, Butanole; Aether und ätherartige Verbindungen wie beispielsweise Dialkyläther, wie Diäthyläther, Diisopropyläther; Ketone wie beispielsweise Aceton, Diäthylketon, Methyläthylketon oder Gemische solcher Lösungsmittel untereinander.

Die Umsetzung gemäss Verfahrensvariante a) wird zweckmässigerweise bei erhöhter Temperatur, bevorzugt im Bereich von 100° bis 200 °C, insbesondere bei 140° bis 160 °C durchgeführt.

Das bei dieser Umsetzung entstehende Methanthiol lässt sich aus dem Reaktionsgemisch leicht auf an sich bekannte Weise entfernen, beispielsweise durch Einleitung in Natriumhypochlorit.

Bei der Umsetzung gemäss Verfahrensvariante b) geht man zweckmässigerweise so vor, dass man 3-Methylamino-4-amino-6-tert.butyl-4,5-dihydro-1,2,4-triazin-5-on in Gegenwart eines Transferkatalysators mit Dimethylsulfat oder einem Methylhalogenid umsetzt. Es ist vorteilhaft, die Umsetzung in Gegenwart eines organischen Lösungsmittels, einer starken Base und eines Transferkatalysators vorzunehmen, wobei es besonders günstig ist, in eine Mischung aus einem organischen Lösungsmittel und einer starken Base 3-Methylamino-4-amino-6-tert.butyl-4,5-dihydro-1,2,4-triazin-5-on einzutragen und dieses Gemisch mit a) Dimethylsulfat oder insbesondere einem Methylhalogenid und b) einem Phasentransferkatalysator, insbesondere einem quaternären Ammoniumsalz oder -hydroxid oder einem Phosphoniumsalz, zu versetzen.

Als starke Basen kommen beispielsweise Alkalimetallhydroxide, wie Natrium- oder Kaliumhydroxid, oder Alkali- oder Erdalkalicarbonate in Betracht.

Als organische Lösungsmittel eignen sich insbesondere die vorstehend unter Verfahrensvariante a) genannten.

Unter Methylhalogeniden sind z. B. Methylchlorid, Methylbromid und insbesondere Methyljodid zu verstehen.

Als Ammoniumsalze oder -hydroxide kommen vorzugsweise solche der Gruppe Benzyltrialkylammonium- oder Tetraalkylammonium-hydroxid, -bisulfat oder -halogenid in Betracht, in denen die Alkylreste zweckmässigerweise 1 bis 4 Kohlenstoffatome enthalten, wie beispielsweise Benzyltriäthylammoniumchlorid, Tetra-n-butylammonium-hydroxid, Benzyltrimethylammoniumchlorid. Besonders geeignet ist ein Tetraalkylammoniumhalogenid, insbesondere Tetra-n-butylammonium-bromid.

Beispiele für Phosphoniumsalze sind Tributylhexadecylphosphonium-bromid, Aethyltriphenylphosphonium-bromid, Tetraphenylphosphonium-chlorid, Benzyltriphenylphosphonium-jodid, Triphenyl-n-propylphosphonium-bromid und Tetrabutylphosphonium-chlorid.

Die Umsetzung gemäss Verfahrensvariante b) lässt sich in einem breiteren Temperaturbereich durchführen, wobei jedoch ein Temperaturbereich zwischen 10° und 40 °C als besonders vorteilhaft anzusehen ist, und insbesondere ein Bereich von 20° bis 25 °C.

Das in der Verfahrensvariante a) verwendete 3-Methylthio-4-methylamino-6-tert.butyl-4,5-dihydro-1,2,4-triazin-5-on und seine Herstellung sind in Z. Naturforsch. C, Biosci., 75, 30C, p. 499-504 und Z. Naturforsch. B, Anorg. Chem., Org. Chem., 76, 31B, p. 1 122-6 beschrieben.

3-Methylthio-4-methylamino-6-tert.butyl-4,5-dihydro-1,2,4-triazin-5-on lässt sich unter den gleichen Verfahrensbedingungen, wie sie für die Umsetzung gemäss Verfahrensvariante b) beschrieben sind, durch Umsetzung von 3-Methylthio-4-amino-6-tert.butyl-4,5-dihydro-1,2,4-triazin-5-on mit einem Methylhalogenid oder Dimethylsulfat herstellen.

2

Zur Applikation als Herbizid wird 3,4-Di-(methylamino)-6-tert.butyl-4,5-dihydro-1,2,4-triazin-5-on in unveränderter Form oder vorzugsweise zusammen mit den in der Formulierungstechnik üblichen Hilfsmitteln eingesetzt und wird daher z. B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt.

Die Formulierungen, d. h., die den Wirkstoff 3,4-Di-(methylamino)-6-tert.butyl-4,5-dihydro-1,2,4-triazin-5-on und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen des Wirkstoffs mit Streckmitteln, wie z. B. mit Lösungsmitteln, festen Trägerstoffen, und gegebenenfalls oberflächenaktiven Verbindungen (Tensiden).

Als Lösungsmittel können in Frage kommen : Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl ; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus können eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z. B. die Na- oder K-Salze der Oel- oder Stearinsäure, oder von natürlichen Fettsäuregemischen genannt, die z. B. aus Kokosnuss- oder Talgöl gewonnen werden können. Ferner sind auch die Fettsäuremethyltaurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxyd-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z. B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Aethylenoxyd-Adduktes oder Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindung enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxydaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z. B. das Stearyltrimethylammoniumchlorid oder das Benzyldi(2-chloräthyl)äthylammoniumbromid.

Die in der Formulierungstechnik gebräuchlichen Tenside sind u. a. in folgenden Publikationen beschrieben :

« Mc Cutcheon's Detergents and Emulsifiers Annual » MC Publishing
Corp., Ridgewood New Jersey, 1980
Sisley and Wood, « Encyclopedia of Surface Active Agents »,
Chemical Publishing Co., Inc. New York, 1964.
Stache, H. « Tensid-Taschenbuch » Carl Hanser Verlag, München/Wien, 1981.

Die agrochemischen Zubereitungen enthalten in der Regel 0,1 bis 99 %, insbesondere 0,1 bis 95 %, Wirkstoff, 99,9 bis 1 %, insbesondere 99,8 bis 5 % eines festen oder flüssigen Zusatzstoffes und 0 bis 25 %, insbesondere 0,1 bis 25 % eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Das 3,4-Di-(methylamino)-6-tert.butyl-4,5-dihydro-1,2,4-triazin-5-on zeigt eine ausgeprägte herbizide Aktivität. Es ist zur Bekämpfung sowohl monokotyler wie dikotyler Unkräuter geeignet, und zwar sowohl bei der Anwendung im Vorauflaufverfahren (pre-emergent) wie im Nachauflaufverfahren (post-emergent).

Besonders vorteilhaft lassen sich 3,4-Di-(methylamino)-6-tert.butyl-4,5-dihydro-1,2,4-triazin-5-on oder es enthaltende Mittel zur selektiven Bekämpfung von Unkräutern in Nutzpflanzenkulturen einsetzen, wie beispielsweise in Kulturen von Getreide, Mais, Soja und insbesondere Zuckerrohr. Beim Einsatz in Zuckerrohr kann es zu einer beachtlichen Steigerung des Zuckergehalts kommen.

Die Aufwandmengen, in denen 3,4-Di-(methylamino)-6-tert.butyl-4,5-dihydro-1,2,4-triazin-5-on anzuwenden ist, hängen von den jeweiligen Gegebenheiten wie insbesondere vom Pflanzenbewuchs, der Bodenbeschaffenheit, den Witterungsbedingungen und dem Zeitpunkt der Applikation ab. Als zweckmässig erweisen sich im allgemeinen Aufwandmengen von 30 bis 2 000 g/Hektar.

## Beispiel 1

### 3-Methylthio-4-methylamino-6-tert.butyl-4,5-dihydro-1,2,4-triazin-5-on (Zwischenprodukt)

321,0 g (1,5 M) 3-Methylthio-4-amino-6-tert.butyl-4,5-dihydro-1,2,4-triazin-5-on werden bei Raumtemperatur in eine Mischung von 600 ml 50 %iger Natronlauge und 600 ml Toluol eingetragen. Bei Raumtemperatur gibt man anschliessend 244 ml (3,75 M) Methyljodid und 50 g Tetrabutylammoniumbromid auf einmal zu. Durch die leicht exotherme Reaktion steigt die Temperatur auf 40 °C. Man lässt das Reaktionsgemisch 30 Minuten lang ausrühren und giesst es in einen Scheidetrichter. Dann trennt man soviel der anorganischen Phase wie möglich ab. Den ganzen Rest gibt man auf 10 Liter Eis/Wasser und rührt intensiv. Anschliessend filtriert man das auskristallisierte, weisse Produkt ab und wäscht solange mit Wasser nach, bis das Filtrat neutral ist. Nach dem Trocken über Phosphorpentoxyd erhält man 336,2 g (98,2 % d. Th.) 3-Methylthio-4-methylamino-6-tert.butyl-4,5-dihydro-1,2,4-triazin-5-on. Smp. 132 bis 133 °C.

## Beispiel 2

### 3,4-Di-(methylamino)-6-tert.butyl-4,5-dihydro-1,2,4-triazin-5-on (Endprodukt)

Ein Gemisch von 233 g (1,02 M) 3-Methylthio-4-methylamino-6-tert.butyl-4,5-dihydro-1,2,4-triazin-5-on, 37,8 g (1,22 M) Methylamin und 280 ml 2-Propanol wird im Autoklaven 4 Stunden auf 150 °C erhitzt. Nach dem Abkühlen bläst man mit Stickstoff das entstandene Methanthiol zur Vernichtung durch eine Natriumhypochlorit-Lösung aus. Die leicht trübe Lösung filtriert man durch eine kleine Schicht Kieselerde ab und dampft die nun klare, gelbe Lösung im Vakuum bei 50 °C ein. Das zurückbleibende, gelborange, klare Oel nimmt man in heissem Essigester auf und erhält nach dem Abkühlen 203,5 g (94,4 % d. Th.) 3,4-Di-(methylamino)-6-tert.butyl-4,5-dihydro-1,2,4-triazin-5-on. Smp. nach Umkristallisation aus Methylenchlorid 163 bis 165 °C.

Formulierungsbeispiele für 3,4-Di-(methylamino)-6-tert.butyl-4,5-dihydro-1,2,4-triazin-5-on

(% = Gewichtsprozent)

| 3. Spritzpulver | a) | b) | c) |
|---|---|---|---|
| 3,4-Di-(methylamino)-6-tert.butyl-4,5-dihydro-1,2,4-triazin-5-on | 25 % | 50 % | 75 % |

4

(Fortsetzung)

| | a) | b) | c) |
|---|---|---|---|
| Na-Ligninsulfonat | 5 % | 5 % | — |
| Na-Laurylsulfat | 3 % | — | 5 % |
| Na-Diisobutylnaphthalinsulfonat | — | 6 % | 10 % |
| Octylphenolpolyäthylenglykoläther (7-8 Mol AeO) | — | 2 % | — |
| Hochdisperse Kieselsäure | 5 % | 10 % | 10 % |
| Kaolin | 62 % | 27 % | — |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

### 4. Emulsions-Konzentrat

| | |
|---|---|
| 3,4-Di-(methylamino)-6-tert.butyl-4,5-dihydro-1,2,4-triazin-5-on | 10 % |
| Octylphenolpolyäthylenglykoläther (4-5 Mol AeO) | 3 % |
| Ca-Dodecylbenzolsulfonat | 3 % |
| Ricinusölpolyglykoläther (35 Mol AeO) | 4 % |
| Dimethylformamid | 20 % |
| Cyclohexanon | 20 % |
| Xylolgemisch | 40 % |

Aus diesem Konzentrat können durch Verdünnen mit Wasser Emulsionen jeder gewünschten Konzentration hergestellt werden.

### 5. Stäubemittel

| | a) | b) |
|---|---|---|
| 3,4-Di-(methylamino)-6-tert.butyl-4,5-dihydro-1,2,4-triazin-5-on | 5 % | 8 % |
| Talkum | 95 % | — |
| Kaolin | — | 92 % |

Man erhält anwendungsfertige Stäubemittel, indem der Wirkstoff mit den Trägerstoffen vermischt und auf einer geeigneten Mühle vermahlen wird.

### 6. Extruder-Granulat

| | |
|---|---|
| 3,4-Di-(methylamino)-6-tert.butyl-4,5-dihydro-1,2,4-triazin-5-on | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

### 7. Umhüllung-Granulat

| | |
|---|---|
| 3,4-Di-(methylamino)-6-tert.butyl-4,5-dihydro-1,2,4-triazin-5-on | 3 % |
| Polyäthylenglykol (MG 200) | 3 % |
| Kaolin, gekörnt | 94 % |

Der fein gemahlene Wirkstoff wird in einem Mischer auf das mit Polyäthylenglykol angefeuchtete, gekörnte Kaolin gleichmässig aufgetragen. Auf diese Weise erhält man staubfreie Umhüllungs-Granulate.

### 8. Suspensions-Konzentrat

| | |
|---|---|
| 3,4-Di-(methylamino)-6-tert.butyl 4,5-dihydro-1,2,4-triazin-5-on | 40 % |
| Aethylenglykol | 10 % |
| Nonylphenolpolyäthylenglykoläther (15 Mol AeO) | 6 % |

| Na-Ligninsulfonat | 10 % |
| Carboxymethylcellulose | 1 % |
| 37 %-ige wässrige Formaldehyd-Lösung | 0,2 % |
| Silikonöl in Form einer 75 %-igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem duch Verdünnen mit Wasser Suspensionen jeder gewünschten Konzentration hergestellt werden können.

## Beispiel 9

Pre-emergente Herbizid-Wirkung

Im Gewächshaus wird unmittelbar nach der Einsaat der Versuchspflanzen in Töpfe von 11 cm Durchmesser die Erdoberfläche mit einer wässrigen Dispersion von 3,4-Di-(methylamino)-6-tert.butyl-4,5-dihydro-1,2,4-triazin-5-on, erhalten aus einem 25 %igen Spritzpulver, behandelt. Es werden Konzentrationen von 30 g/Hektar angewendet. Die angesäten Töpfe werden im Gewächshaus bei 20 bis 24 °C und 50 bis 70 % relativer Luftfeuchtigkeit gehalten. Der Versuch wird nach 3 Wochen ausgewertet und die Resultate werden nach folgender Notenskala boniert :

1 = Pflanzen nicht gekeimt oder total abgestorben
2-3 = sehr starke Wirkung
4-6 = mittlere Wirkung
7-8 = geringe Wirkung
9 = keine Wirkung (wie unbehandelte Kontrolle)

| Weizen | 7 |
| Mais | 9 |
| Soja | 8 |
| Baumwolle | 8 |
| Zuckerrübe | 7 |
| Abutilon | 1 |
| Chenopodium | 3 |
| Ipomoea | 2 |
| Sinapis | 4 |

## Beispiel 10

Post-emergente Herbizid-Wirkung

Im Gewächshaus werden in Töpfen von 11 cm Durchmesser die Pflanzen Mais, Trockenreis, Soja, Avena fatua, Alopecurus, Abutilon, Xanthium, Sinapis und Viola tricolor gezogen, bis sie das 3-6 Blatt-Stadium erreicht haben, was nach ca. 2 Wochen der Fall ist. Dann werden sie mit einer wässrigen Wirkstoffemulsion in einer Dosierung von 60 g Wirksubstanz pro Hektar gespritzt und dann bei 20-24 °C und 45-60 % relativer Luftfeuchtigkeit gehalten. 15 Tage nach der Behandlung wird der Versuch ausgewertet und das Ergebnis wie im Vorauflauf-Versuch nach derselben Notenskala ausgewertet. Die Resultate sind wie folgt :

| Mais | 9 |
| Trockenreis | 9 |
| Soja | 7 |
| Avena fatua | 2 |
| Alopecurus | 1 |
| Abutilon | 1 |
| Xanthium | 1 |
| Sinapis | 1 |
| Viola tricolor | 3 |

## Beispiel 11

Selektiv-herbizide Wirkung gegen Ipomoea in Zuckerrohr

Freilandparzellen von 8 m², welche Samen der Unkrautpflanze Ipomoea sp. enthalten, werden mit Zuckerrohr bepflanzt. Wenn die Zuckerrohrpflanzen 1 bis 4 Blätter und eine Wuchshöhe von 10 bis 30 cm aufweisen, wird 3,4-Di-(methylamino)-6-tert.butyl-4,5-dihydro-1,2,4-triazin-5-on noch vor dem Auflaufen des Unkrauts als wässrige Spritzbrühe in einer Menge, welche 400 l/ha entspricht, auf Boden und Pflanzen appliziert. Die Resultate sind in Tabelle 1 zusammengefasst.

Tabelle 1

| kg Wirkstoff pro Hektar | Wirkung gegen Ipomoea sp. in % der Kontrolle; Tage nach Applikation | | | | | Nebenwirkungen auf Zuckerrohr in % der Kontrolle; Tage nach Applikation | | | | |
|---|---|---|---|---|---|---|---|---|---|---|
| | 11 | 26 | 40 | 70 | 101 | 11 V | 26 V | 40 W | 70 W | 101 W |
| 1,0 | 50 | 96 | 98 | 98 | 95 | 0 | 0 | 0 | 0 | 0 |
| 1,5 | 70 | 100 | 100 | 100 | 100 | 0 | 0 | 0 | 0 | 0 |
| 2,0 | 100 | 98 | 96 | 97 | 95 | 0 | 0 | 0 | 0 | 0 |

V = Verfärbung (Welkesymptome)
W = Wuchshemmung

In den folgenden Beispielen 12 bis 14 werden die nachstehend angegebenen Testsubstanzen eingesetzt :

A) = 3,4-Di-(methylamino)-6-tert.butyl-4,5-dihydro-1,2,4-triazin-5-on gemäss vorliegender Erfindung
B) = 3-Methylamino-4-amino-6-tert.butyl-4,5-dihydro-1,2,4-triazin-5-on gemäss US-PS 3 671 523 als Vergleichssubstanz ;
C) = 3-Methylthio-4-amino-6-tert.butyl-4,5-dihydro-1,2,4-triazin-5-on gemäss US-PS 3 671 523 als Vergleichssubstanz.

Die Testsubstanzen werden als wässrige Spritzbrühen appliziert, welche aus 25 %igem Spritzpulver bei den Verbindungen A) und B) und aus 70 %igem Spritzpulver bei der Verbindung C) erhalten worden sind.

Beispiel 12

Selektiv-herbizide Wirkung gegen Panicum maximum in Zuckerrohr

Auf Freilandparzellen von 8 m² lässt man Zuckerrohrpflanzen und Unkrautpflanzen der Spezies Panicum maximum heranwachsen. Wenn die Zuckerrohrpflanzen 2 bis 5 Blätter und eine Wuchshöhe von 20 bis 45 cm und die Unkrautpflanzen 1 bis 4 Blätter und eine Wuchshöhe von 1 bis 8 cm aufweisen, werden die Testsubstanzen als wässrige Spritzbrühen in einer Menge, welche 400 l/ha entspricht, auf die Pflanzen appliziert. Die Resultate zeigt Tabelle 2.

Tabelle 2

| Verbindung | kg Wirkstoff pro Hektar | Wirkung gegen Panicum maximum in % der Kontrolle; Tage nach Applikation | | | | Phytotoxizität auf Zuckerrohr 42 Tage nach Applikation |
|---|---|---|---|---|---|---|
| | | 8 | 21 | 42 | 61 | |
| A | 1,0 | 85 | 96 | 98 | 97 | 0 |
| B | 1,0 | 10 | 0 | 0 | 0 | 0 |
| C | 1,0 | 10 | 0 | 0 | 0 | 0 |
| A | 1,5 | 85 | 98 | 98 | 100 | 0 |
| B | 1,5 | 0 | 0 | 0 | 0 | 0 |
| C | 1,5 | 20 | 0 | 0 | 0 | 0 |
| A | 2,0 | 98 | 99 | 100 | 100 | 0 |
| B | 2,0 | 20 | 30 | 20 | 30 | 0 |
| C | 2,0 | 40 | 30 | 20 | 20 | 0 |

# 0 114 783

## Beispiel 13

### Selektiv-herbizide Wirkung gegen Cenchrus echinatus in Zuckerrohr

Freilandparzellen von 8 m², welche Samen und Keimlinge des Unkrauts Cenchrus echinatus enthalten, werden mit Zuckerrohr bepflanzt. Wenn die Zuckerrohrpflanzen 0 bis 2 Blätter und ein Wuchshöhe von 0 bis 15 cm und die Unkrautpflanzen 1 bis 3 Blätter und eine Wuchshöhe von 0 bis 4 cm aufweisen, werden die Testsubstanzen als wässrige Spritzbrühen in einer Menge, welche 400 l/ha entspricht, auf Boden und Pflanzen appliziert. Die Resultate sind in Tabelle 3 wiedergegeben.

### Tabelle 3

| Ver-bin-dung | kg Wirk-stoff pro Hektar | Wirkung gegen Cenchrus echinatus in % der Kon-trolle; Tage nach Applikation | | | | | Nebenwirkungen auf Zuk-kerrohr in % der Kon-trolle; Tage nach Applikation | | |
|---|---|---|---|---|---|---|---|---|---|
| | | 12 | 26 | 42 | 60 | 77 | 12 B | 60 W | 77 W |
| A | 1,0 | 96 | 95 | 96 | 85 | 80 | 0 | 0 | 0 |
| B | 1,0 | 85 | 50 | 40 | 10 | 0 | 0 | 0 | 0 |
| C | 1,0 | 80 | 60 | ·50 | 10 | 0 | 0 | 0 | 0 |
| A | 1,5 | 97 | 98 | 98 | 90 | 90 | 0 | 0 | 0 |
| B | 1,5 | 95 | 70 | 60 | 30 | 20 | 0 | 0 | 0 |
| C | 1,5 | 92 | 70 | 60 | 20 | 20 | 0 | 0 | 0 |
| A | 2,0 | 98 | 98 | 98 | 95 | 94 | 0 | 0 | 0 |
| B | 2,0 | 97 | 70 | 70 | 30 | 20 | 0 | 0 | 0 |
| C | 2,0 | 98 | 85 | 80 | 30 | 20 | 0 | 0 | 0 |

B = Verbrennungen
W = Wuchshemmung

## Beispiel 14

### Selektiv-herbizide Wirkung gegen Brachiaria decumbens in Zuckerrohr

Auf Freilandparzellen von 8 m² lässt man Zuckerrohrpflanzen und Unkrautpflanzen der Spezies Brachiaria decumbers heranwachsen. Wenn die Zuckerrohrpflanzen 3 bis 6 Blätter und eine Wuchshöhe von 30 bis 70 cm und die Unkrautpflanzen 2 bis 4 Blätter und eine Wuchshöhe von 3 bis 10 cm aufweisen, werden die Testsubstanzen als wässrige Spritzbrühen in einer Menge, welche 400 l/ha entspricht, auf die Pflanzen appliziert. Die Resultate sind in Tabelle 4 zusammengefasst.

### Tabelle 4

| Ver-bin-dung | kg Wirk-stoff pro Hektar | Wirkung gegen Brachiaria decumbens in % der Kon-trolle; Tage nach Applikation | | | Nebenwirkungen auf Zuk-kerrohr in % der Kon-trolle; Tage nach Applikation | |
|---|---|---|---|---|---|---|
| | | 21 | ·42 | 64 · | 7 V | 42 Ph |
| A | 1,0 | 85 | 90 | 94 | 0 | 0 |
| B | 1,0 | 50 | 40 | 70 | 0 | 0 |
| C | 1,0 | · 70 | 50 | 70 | 0 | 0 |
| A | 2,0 | 92 | 92 | 97 | 0 | 0 |
| B | 2,0 | 60 | 40 | 70 | 0 | 0 |
| C | 2,0 | 85 | 80 | 94 | 0 | 0 |
| A | 4,0 | 97 | 96 | 100 | 0 | 0 |
| B | 4,0 | 70 | 70 | 85 | 0 | 0 |
| C | 4,0 | 94 | 95 | 98 | 0 | 0 |

V = Verfärbung (Weikesymptome)
Ph = Phytotoxizität

8

**0 114 783**

1. 3,4-Di-(methylamino)-6-tert.butyl-4,5-dihydro-1,2,4-triazin-5-on.

2. Verfahren zur Herstellung von 3,4-Di-(methylamino)-6-tert.butyl-4,5-dihydro-1,2,4-triazin-5-on, dadurch gekennzeichnet, dass man 3-Methylthio-4-methylamino-6-tert.butyl-4,5-dihydro-1,2,4-triazin-5-on mit Methylamin umsetzt.

3. Verfahren nach Anspruch 2, dadurch gekennzeichnet, dass man 3-Methylthio-4-methylamino-6-tert.butyl-4,5-dihydro-1,2,4-triazin-5-on, welches durch Umsetzung von 3-Methylthio-4-amino-6-tert.butyl-4,5-dihydro-1,2,4-triazin-5-on mit einem Methylhalogenid oder mit Dimethylsulfat hergestellt wird, mit Methylamin umsetzt.

4. Verfahren nach Anspruch 2, dadurch gekennzeichnet, dass man die Umsetzung bei einer Temperatur im Bereich von 100 bis 200 °C durchführt.

5. Verfahren zur Herstellung von 3,4-Di-(methylamino)-6-tert.butyl-4,5-dihydro-1,2,4-triazin-5-on, dadurch gekennzeichnet, dass man 3-Methylamino-4-amino-6-tert.butyl-4,5-dihydro-1,2,4-triazin-5-on mit einem Methylhalogenid oder mit Dimethylsulfat umsetzt.

6. Verfahren nach Anspruch 5, dadurch gekennzeichnet, dass man 3-Methylamino-4-amino-6-tert.butyl-4,5-dihydro-1,2,4-triazin-5-on in Gegenwart eines Phasentransferkatalysators mit einem Methylhalogenid umsetzt.

7. Verfahren nach Anspruch 6, dadurch gekennzeichnet, dass man 3-Methylamino-4-amino-6-tert.butyl-4,5-dihydro-1,2,4-triazin-5-on in Gegenwart eines organischen Lösungsmittels, einer starken Base und eines Phasentransferkatalysators mit einem Methylhalogenid umsetzt.

8. Verfahren nach Anspruch 6, dadurch gekennzeichnet, dass man als Phasentransferkatalysator ein quaternäres Ammoniumsalz oder -hydroxid oder ein Phosphoniumsalz verwendet.

9. Verfahren nach Anspruch 8, dadurch gekennzeichnet, dass man als Phasentransferkatalysator ein Benzyltrialkylammoniumhydroxid, ein Benzyltrialkylammoniumbisulfat, ein Benzyltrialkylammoniumhalogenid, ein Tetraalkylammoniumhydroxid, ein Tetraalkylammoniumbisulfat oder ein Tetraalkylammoniumhalogenid verwendet.

10. Verfahren nach Anspruch 9, dadurch gekennzeichnet, dass man als Phasentransferkatalysator ein Tetraalkylammoniumhalogenid verwendet.

11. Mittel zur Bekämpfung von unerwünschtem Pflanzenwuchs, dadurch gekennzeichnet, dass es neben Träger- und/oder anderen Zusatzstoffen als aktive Komponente 3,4-Di-(methylamino)-6-tert.butyl-4,5-dihydro-1,2,4-triazin-5-on enthält.

12. Mittel nach Anspruch 11, dadurch gekennzeichnet, dass es 0,1 bis 99 Gewichtsprozent 3,4-Di-(methylamino)-6-tert.butyl-4,5-dihydro-1,2,4-triazin-5-on, 99,9 bis 1 Gewichtsprozent an einem oder mehreren Zusatzstoffen und 0 bis 25 Gewichtsprozent eines Tensides enthält.

13. Mittel nach Anspruch 12, dadurch gekennzeichnet, dass es 0,1 bis 95 Gewichtsprozent 3,4-Di-(methylamino)-6-tert.butyl-4,5-dihydro-1,2,4-triazin-5-on, 5 bis 99,8 Gewichtsprozent an einem oder mehreren Zusatzstoffen und 0,1 bis 25 Gewichtsprozent eines Tensides enthält.

14. Verwendung von 3,4-Di(methylamino)-6-tert.butyl-4,5-dihydro-1,2,4-triazin-5-on zur Bekämpfung von unerwünschtem Pflanzenwuchs.

15. Verwendung nach Anspruch 14 zur Bekämpfung von Unkräutern in Kulturpflanzenbeständen.

16. Verwendung nach Anspruch 15 zur Bekämpfung von Unkräutern in Kulturen von Getreide, Mais, Soja und Zuckerrohr.

17. Verwendung nach Anspruch 16 zur Bekämpfung von Unkräutern in Kulturen von Zuckerrohr.

18. Verwendung nach Anspruch 15 zur Bekämpfung monokotyler Unkräuter.

19. Verwendung nach Anspruch 15 zur Bekämpfung dikotyler Unkräuter.

20. Verfahren zur Bekämpfung von Unkräutern in Kulturpflanzenbeständen, dadurch gekennzeichnet, dass man 3,4-Di-(methylamino)-6-tert.butyl-4,5-dihydro-1,2,4-triazin-5-on oder ein Mittel, welches diese Verbindung enthält, auf die Kulturen appliziert.

21. Verfahren nach Anspruch 20, dadurch gekennzeichnet, dass man 3,4-Di-(methylamino)-6-tert. butyl-4,5-dihydro-1,2,4-triazin-5-on oder ein Mittel, welches diese Verbindung enthält, in einer Aufwandmenge, welche 30 bis 2 000 g 3,4-Di-(methylamino)-6-tert.butyl-4,5-dihydro-1,2,4-triazin-5-on pro Hektar entspricht, appliziert.

**Patentansprüche** (für den Vertragsstaat AT)

1. Mittel zur Bekämpfung von unerwünschtem Pflanzenwuchs, dadurch gekennzeichnet, dass es neben Träger- und/oder anderen Zusatzstoffen als aktive Komponente 3,4-Di-(methylamino)-6-tert.butyl-4,5-dihydro-1,2,4-triazin-5-on enthält.

2. Mittel nach Anspruch 1, dadurch gekennzeichnet, dass es 0,1 bis 99 Gewichtsprozent 3,4-Di-(methylamino)-6-tert.butyl-4,5-dihydro-1,2,4-triazin-5-on, 99,9 bis 1 Gewichtsprozent an einem oder mehreren Zusatzstoffen und 0 bis 25 Gewichtsprozent eines Tensides enthält.

3. Mittel nach Anspruch 2, dadurch gekennzeichnet, dass es 0,1 bis 95 Gewichtsprozent 3,4-Di-(methylamino)-6-tert.butyl-4,5-dihydro-1,2,4-triazin-5-on, 5 bis 99,8 Gewichtsprozent an einem oder mehreren Zusatzstoffen und 0,1 bis 25 Gewichtsprozent eines Tensides enthält.

4. Verfahren zur Herstellung von 3,4-Di-(methylamino)-6-tert.butyl-4,5-dihydro-1,2,4-triazin-5-on, dadurch gekennzeichnet, dass man 3-Methylthio-4-methylamino-6-tert.butyl-4,5-dihydro-1,2,4-triazin-5-on mit Methylamin umsetzt.

5. Verfahren nach Anspruch 4, dadurch gekennzeichnet, dass man 3-Methylthio-4-methylamino-6-tert.butyl-4,5-dihydro-1,2,4-triazin-5-on, welches durch Umsetzung von 3-Methylthio-4-amino-6-tert.butyl-4,5-dihydro-1,2,4-triazin-5-on mit einem Methylhalogenid oder mit Dimethylsulfat hergestellt wird, mit Methylamin umsetzt.

6. Verfahren nach Anspruch 4, dadurch gekennzeichnet, dass man die Umsetzung bei einer Temperatur im Bereich von 100 bis 200 °C durchführt.

7. Verfahren zur Herstellung von 3,4-Di-(methylamino)-6-tert.butyl-4,5-dihydro-1,2,4-triazin-5-on, dadurch gekennzeichnet, dass man 3-Methylamino-4-amino-6-tert.butyl-4,5-dihydro-1,2,4-triazin-5-on mit einem Methylhalogenid oder mit Dimethylsulfat umsetzt.

8. Verfahren nach Anspruch 7, dadurch gekennzeichnet, dass man 3-Methylamino-4-amino-6-tert.butyl-4,5-dihydro-1,2,4-triazin-5-on in Gegenwart eines Phasentransferkatalysators mit einem Methylhalogenid umsetzt.

9. Verfahren nach Anspruch 8, dadurch gekennzeichnet, dass man 3-Methylamino-4-amino-6-tert.butyl-4,5-dihydro-1,2,4-triazin-5-on in Gegenwart eines organischen Lösungsmittels, einer starken Base und eines Phasentransferkatalysators mit einem Methylhalogenid umsetzt.

10. Verfahren nach Anspruch 8, dadurch gekennzeichnet, dass man als Phasentransferkatalysator ein quarternäres Ammoniumsalz oder -hydroxid oder ein Phosphoniumsalz verwendet.

11. Verfahren nach Anspruch 10, dadurch gekennzeichnet, dass man als Phasentransferkatalysator ein Benzyltrialkylammoniumhydroxid, ein Benzyltrialkylammoniumbisulfat, ein Benzyltrialkylammoniumhalogenid, ein Tetraalkylammoniumhydroxid, ein Tetraalkylammoniumbisulfat oder ein Tetraalkylammoniumhalogenid verwendet.

12. Verfahren nach Anspruch 11, dadurch gekennzeichnet, dass man als Phasentransferkatalysator ein Tetraalkylammoniumhalogenid verwendet.

13. Verfahren zur Herstellung eines Mittels zur Bekämpfung von Unkräutern, dadurch gekenzeichnet, dass man als aktive Komponente 3,4-Di-(methylamino)-6-tert.butyl-4,5-dihydro-1,2,4-triazin-5-on mit inertem Träger- und/oder anderen Zusatzstoffen vereinigt.

14. Verwendung von 3,4-Di-(methylamino)-6-tert.butyl-4,5-dihydro-1,2,4-triazin-5-on zur Bekämpfung von unerwünschtem Pflanzenwuchs.

15. Verwendung nach Anspruch 14 zur Bekämpfung von Unkräutern in Kulturpflanzenbeständen.

16. Verwendung nach Anspruch 15 zur Bekämpfung von Unkräutern in Kulturen von Getreide, Mais, Soja und Zuckerrohr.

17. Verwendung nach Anspruch 16 zur Bekämpfung von Unkräutern in Kulturen von Zuckerrohr.

18. Verwendung nach Anspruch 15 zur Bekämpfung monokotyler Unkräuter.

19. Verwendung nach Anspruch 15 zur Bekämpfung dikotyler Unkräuter.

20. Verfahren zur Bekämpfung von Unkräutern in Kulturpflanzenbeständen, dadurch gekennzeichnet, dass man 3,4-Di-(methylamino)-6-tert.-butyl-4,5-dihydro-1,2,4-triazin-5-on oder ein Mittel, welches diese Verbindung enthält, auf die Kulturen appliziert.

21. Verfahren nach Anspruch 20, dadurch gekennzeichnet, dass man 3,4-Di-(methylamino)-6-tert.butyl-4,5-dihydro-1,2,4-triazin-5-on oder ein Mittel, welches diese Verbindung enthält, in einer Aufwandmenge, welche 30 bis 2 000 g 3,4-Di-(methylamino)-6-tert.butyl-4,5-dihydro-1,2,4-triazin-5-on pro Hektar entspricht, appliziert.


**Claims** (for the Contracting States : DE, FR, CH, LI, IT, NL, BE, SE)

1. 3,4-Di(methylamino)-6-tert-butyl-4,5-dihydro-1,2,4-triazin-5-one.

2. A process for the preparation of 3,4-di(methylamino)-6-tert-butyl-4,5-dihydro-1,2,4-triazin-5-one, wherein 3-methylthio-4-methylamino-6-tert-butyl-4,5-dihydro-1,2,4-triazin-5-one is reacted with methylamine.

3. A process according to claim 2, wherein 3-methylthio-4-methylamino-6-tert-butyl-4,5-dihydro-1,2,4-triazin-5-one, which is prepared by reacting 3-methylthio-4-amino-6-tert-butyl-4,5-dihydro-1,2,4-triazin-5-one with a methyl halide or with dimethyl sulfate, is reacted with methylamine.

4. A process according to claim 2, wherein the reaction is carried out at a temperature in the range from 100° to 200 °C.

5. A process for the preparation of 3,4-di(methylamino)-6-tert-butyl-4,5-dihydro-1,2,4-triazin-5-one, wherein 3-methylamino-4-amino-6-tert-butyl-4,5-dihydro-1,2,4-triazin-5-one is reacted with a methyl halide or with dimethyl sulfate.

6. A process according to claim 5, wherein 3-methylamino-4-amino-6-tert-butyl-4,5-dihydro-1,2,4-triazin-5-one is reacted with a methyl halide in the presence of a phase transfer catalyst.

7. A process according to claim 6, wherein 3-methylamino-4-amino-6-tert-butyl-4,5-dihydro-1,2,4-triazin-5-one is reacted with a methyl halide in the presence of an organic solvent, a strong base and a phase transfer catalyst.

8. A process according to claim 6, wherein the phase transfer catalyst is a quaternary ammonium salt or ammonium hydroxide or a phosphonium salt.

9. A process according to claim 8, wherein the phase transfer catalyst is selected from the group comprising: a benzyltrialkylammonium hydroxide, a benzyltrialkylammonium bisulfate, a benzyltrialkylammonium halide, a tetraalkylammonium hydroxide, a tetraalkylammonium bisulfate and a tetraalkylammonium halide.

10. A process according to claim 9, wherein the phase transfer catalyst is a tetraalkylammonium halide.

11. A composition for controlling undesired plant growth, which composition contains as active ingredient 3,4-di-(methylamino)-6-tert-butyl-4,5-dihydro-1,2,4-triazin-5-one, together with carriers and/or other additives.

12. A composition according to claim 11, which contains 0.1 to 99 per cent by weight of 3,4-di (methylamino)-6-tert-butyl-4,5-dihydro-1,2,4-triazin-5-one, 99.9 to 1 per cent by weight of one or more additives, and 0 to 25 per cent by weight of a surfactant.

13. A composition according to claim 12, which contains 0.1 to 95 per cent by weight of 3,4-di (methylamino)-6-tert-butyl-4,5-dihydro-1,2,4-triazin-5-one, 5 to 99.8 per cent by weight of one or more additives, and 0.1 to 25 per cent by weight of a surfactant.

14. A method of controlling undesired plant growth, which method comprises the use of 3,4-di(methylamino)-6-tert-butyl-4,5-dihydro-1,2,4-triazin-5-one.

15. A method according to claim 14 for controlling weeds in crops of cultivated plants.

16. A method according to claim 15 for controlling weeds in crops of cereals, maize, soya-bean and sugar cane.

17. A method according to claim 16 for controlling weeds in crops of sugar cane.

18. A method according to claim 15 for controlling monocotyledonous weeds.

19. A method according to claim 15 for controlling dicotyledonous weeds.

20. A method of controlling weeds in crops of cultivated plants, which method comprises applying to the crops 3,4-di(methylamino)-6-tert-butyl-4,5-dihydro-1,2,4-triazin-5-one, or a composition containing said compound.

21. A method according to claim 20, which method comprises applying 3,4-di(methylamino)-6-tert-butyl-4,5-dihydro-1,2,4-triazin-5-one, or a composition containing said compound, at a concentration corresponding to 30 to 2 000 g of 3,4-di(methylamino)-6-tert-butyl-4,5-dihydro-1,2,4-traizin-5-one per hectare.

**Claims** (for the Contracting State AT)

1. A composition for controlling undesired plant growth, which composition contains as active ingredient 3,4-di-(methylamino)-6-tert-butyl-4,5-dihydro-1,2,4-triazin-5-one, together with carriers and/or other additives.

2. A composition according to claim 1, which contains 0.1 to 99 per cent by weight of 3,4-di(methylamino)-6-tert-butyl-4,5-dihydro-1,2,4-triazin-5-one, 99.9 to 1 per cent by weight of one or more additives, and 0 to 25 per cent by weight of a surfactant.

3. A composition according to claim 2, which contains 0.1 to 95 per cent by weight of 3,4-di(methylamino)-6-tert-butyl-4,5-dihydro-1,2,4-triazin-5-one, 5 to 99.8 per cent by weight of one or more additives, and 0.1 to 25 per cent by weight of a surfactant.

4. A process for the preparation of 3,4-di(methylamino)-6-tert-butyl-4,5-dihydro-1,2,4-triazin-5-one, wherein 3-methylthio-4-methylamino-6-tert-butyl-4,5-dihydro-1,2,4-triazin-5-one is reacted with methylamine.

5. A process according to claim 4, wherein 3-methylthio-4-methylamino-6-tert-butyl-4,5-dihydro-1,2,4-triazin-5-one, which is prepared by reacting 3-methylthio-4-amino-6-tert-butyl-4,5-dihydro-1,2,4-triazin-5-one with a methyl halide or with dimethyl sulfate, is reacted with methylamine.

6. A process according to claim 4, wherein the reaction is carried out at a temperature in the range from 100° to 200 °C.

7. A process for the preparation of 3,4-di(methylamino)-6-tert-butyl-4,5-dihydro-1,2,4-triazin-5-one, wherein 3-methylamino-4-amino-6-tert-butyl-4,5-dihydro-1,2,4-triazin-5-one is reacted with a methyl halide or with dimethyl sulfate.

8. A process according to claim 7, wherein 3-methylamino-4-amino-6-tert-butyl-4,5-dihydro-1,2,4-triazin-5-one is reacted with a methyl halide in the presence of a phase transfer catalyst.

9. A process according to claim 8, wherein 3-methylamino-4-amino-6-tert-butyl-4,5-dihydro-1,2,4-triazin-5-one is reacted with a methyl halide in the presence of an organic solvent, a strong base and a phase transfer catalyst.

10. A process according to claim 8, wherein the phase transfer catalyst is a quaternary ammonium salt or ammonium hydroxide or a phosphonium salt.

11. A process according to claim 10, wherein the phase transfer catalyst is selected from the group comprising : a benzyltrialkylammonium hydroxide, a benzyltrialkylammonium bisulfate, a benzyltrialkylammonium halide, a tetraalkylammonium hydroxide, a tetraalkylammonium bisulfate and a tetraalkylammonium halide.

12. A process according to claim 11, wherein the phase transfer catalyst is a tetraalkylammonium halide.

13. A process for the preparation of a composition for controlling weeds, in which process 3,4-di(methylamino)-6-tert-butyl-4,5-dihydro-1,2-triazin-5-one as active ingredient is combined with inert carriers and/or other adjuvants.

14. A method of controlling undesired plant growth, which method comprises the use of 3,4-di(methylamino)-6-tert-butyl-4,5-dihydro-1,2,4-triazin-5-one.

15. A method according to claim 14 for controlling weeds in crops of cultivated plants.

16. A method according to claim 15 for controlling weeds in crops of cereals, maize, soya-bean and sugar cane.

17. A method according to claim 16 for controlling weeds in crops of sugar cane.

18. A method according to claim 15 for controlling monocotyledonous weeds.

19. A method according to claim 15 for controlling dicotyledonous weeds.

20. A method of controlling weeds in crops of cultivated plants, which method comprises applying to the crops 3,4-di(methylamino)-6-tert-butyl-4,5-dihydro-1,2,4-triazin-5-one, or a composition containing said compound.

21. A method according to claim 20, which method comprises applying 3,4-di(methylamino)-6-tert-butyl-4,5-dihydro-1,2,4-triazin-5-one, or a composition containing said compound, at a concentration corresponding to 30 to 2 000 g of 3,4-di(methylamino)-6-tert-butyl-4,5-dihydro-1,2,4-triazin-5-one per hectare.

**Revendications** (pour les Etats contractants : BE, CH, LI, DE, FR, IT, NL, SE)

1. La 3,4-di-(méthylamino)-6-tert.-butyl-4,5-dihydro-1,2,4-triazine-5-one.

2. Procédé de préparation de la 3,4-di-(méthylamino)-6-tert.-butyl-4,5-dihydro-1,2,4-triazine-5-one, caractérisé en ce que l'on fait réagir la 3-méthylthio-4-méthylamino-6-tert.-butyl-4,5-dihydro-1,2,4-triazi-ne-5-one avec la méthylamine.

3. Procédé selon la revendication 2, caractérisé en ce que l'on fait réagir avec la méthylamine une 3-méthylthio-4-méthylamino-6-tert.-butyl-4,5-dihydro-1,2,4-triazine-5-one qui a été préparée par réaction de la 3-méthylthio-4-amino-6-tert.-butyl-4,5-dihydro-1,2,4-triazine-5-one avec un halogénure de méthyle ou le sulfate de diméthyle.

4. Procédé selon la revendication 2, caractérisé en ce que l'on effectue la réaction à une température dans l'intervalle de 100 à 200 °C.

5. Procédé de préparation de la 3,4-di-(méthylamino)-6-tert.-butyl-4,5-dihydro-1,2,4-triazine-5-one, caractérisé en ce que l'on fait réagir la 3-méthylamino-4-amino-6-tert.-butyl-4,5-dihydro-1,2,4-triazine-5-one avec un halogénure de méthyle ou le sulfate de diméthyle.

6. Procédé selon la revendication 5, caractérisé en ce que l'on fait réagir la 3-méthylamino-4-amino-6-tert.-butyl-4,5-dihydro-1,2,4-triazine-5-one avec un halogénure de méthyle en présence d'un catalyseur à transfert de phase.

7. Procédé selon la revendication 6, caractérisé en ce que l'on fait réagir la 3-méthylamino-4-amino-6-tert.-butyl-4,5-dihydro-1,2,4-triazine-5-one avec un halogénure de méthyle en présence d'un solvant organique, d'une base forte et d'un catalyseur à transfert de phase.

8. Procédé selon la revendication 6, caractérisé en ce que l'on utilise en tant que catalyseur à transfert de phase un sel ou hydroxyde d'ammonium quaternaire ou un sel de phosphonium.

9. Procédé selon la revendication 6, caractérisé en ce que l'on utilise en tant que catalyseur à transfert de phase un hydroxyde de benzyltrialkylammonium, un bisulfate de benzyltrialkylammonium, un halogénure de benzyltrialkylammonium, un hydroxyde de tétraalkylammonium, un bisulfate de tétraalkylammonium ou un halogénure de tétraalkylammonium.

10. Procédé selon la revendication 9, caractérisé en ce que l'on utilise en tant que catalyseur à transfert de phase un halogénure de tétraalkylammonium.

11. Produit pour la lutte contre les croissances de végétaux indésirables, caractérisé en ce qu'il contient en tant que composant actif, avec des véhicules et/ou d'autres additifs, la 3,4-di-(méthylamino)-6-tert.-butyl-4,5-dihydro-1,2,4-triazine-5-one.

12. Produit selon la revendication 11, caractérisé en ce qu'il contient de 0,1 à 99 % en poids de 3,4-di-(méthylamino)-6-tert.-butyl-4,5-dihydro-1,2,4-triazine-5-one, de 99,9 à 1 % en poids d'un ou plusieurs additifs et de 0 à 25 % en poids d'un agent tensioactif.

13. Produit selon la revendication 12, caractérisé en ce qu'il contient de 0,1 à 95 % en poids de 3,4-di-(méthylamino)-6-tert.-butyl-4,5-dihydro-1,2,4-triazine-5-one, de 5 à 99,8 % en poids d'un ou plusieurs additifs et de 0,1 à 25 % en poids d'un agent tensioactif.

14. Utilisation de la 3,4-di-(méthylamino)-6-tert.-butyl-4,5-dihydro-1,2,4-triazine-5-one dans la lutte contre les croissances de végétaux indésirables.

15. Utilisation selon la revendication 14, pour la lutte contre les mauvaises herbes dans les cultures de végétaux utiles.

16. Utilisation selon la revendication 15, pour la luttre contre les mauvaises herbes dans les cultures de céréales, de maïs, de soja et de canne à sucre.

17. Utilisation selon la revendication 16, pour la lutte contre les mauvaises herbes dans les cultures de canne à sucre.

18. Utilisation selon la revendication 15, dans la lutte contre les mauvaises herbes monocotylédones.

19. Utilisation selon la revendication 15, dans la lutte contre les mauvaises herbes dicotylédones.

20. Procédé pour combattre les mauvaises herbes dans les cultures de végétaux utiles, caractérisé en ce que l'on applique sur les cultures de la 3,4-di-(méthylamino)-6-tert.-butyl-4,5-dihydro-1,2,4-triazine-5-one ou un produit contenant ce composé.

21. Procédé selon la revendication 20, caractérisé en ce que l'on applique la 3,4-di-(méthylamino)-6-tert.-butyl-4,5-dihydro-1,2,4-triazine-5-one ou un produit contenant ce composé en quantité correspondant à une quantité de 30 à 2 000 g de 3,4-di-(méthylamino)-6-tert.-butyl-4,5-dihydro-1,2,4-triazine-5-one par hectare.

**Revendications** (pour l'Etat contractant AT)

1. Produit pour combattre les cultures de végétaux indésirables, caractérisé en ce qu'il contient en tant que composant actif, avec des véhicules et/ou d'autres additifs, la 3,4-di-(méthylamino)-6-tert.-butyl-4,5-dihydro-1,2,4-triazine-5-one.

2. Produit selon la revendication 1, caractérisé en ce qu'il contient de 0,1 à 99 % en poids de 3,4-di-(méthylamino)-6-tert.-butyl-4,5-dihydro-1,2,4-triazine-5-one, de 99,9 à 1 % en poids d'un ou plusieurs additifs et de 0 à 25 % en poids d'un agent tensioactif.

3. Produit selon la revendication 2, caractérisé en ce qu'il contient de 0,1 à 95 % en poids de 3,4-di-(méthylamino)-6-tert.-butyl-4,5-dihydro-1,2,4-triazine-5-one, de 5 à 99,8 % en poids d'un ou plusieurs additifs et de 0,1 à 25 % en poids d'un agent tensioactif.

4. Procédé de préparation de la 3,4-di-(méthylamino)-6-tert.-butyl-4,5-dihydro-1,2,4-triazine-5-one, caractérisé en ce que l'on fait réagir la 3-méthylthio-4-méthylamino-6-tert.-butyl-4,5-dihydro-1,2,4-triazine-5-one avec la méthylamine.

5. Procédé selon la revendication 4, caractérisé en ce que l'on fait réagir avec la méthylamine une 3-méthylthio-4-méthylamino-6-tert.-butyl-4,5-dihydro-1,2,4-triazine-5-one qui a été préparée par réaction de la 3-méthylthio-4-amino-6-tert.-butyl-4,5-dihydro-1,2,4-triazine-5-one avec un halogénure de méthyle ou du sulfate de diméthyle.

6. Procédé selon la revendication 4, caractérisé en ce que l'on effectue la réaction à une température dans l'intervalle de 100 à 200 °C.

7. Procédé de préparation de la 3,4-di-(méthylamino)-6-tert.-butyl-4,5-dihydro-1,2,4-triazine-5-one, caractérisé en ce que l'on fait réagir la 3-méthylamino-4-amino-6-tert.-butyl-4,5-dihydro-1,2,4-triazine-5-one avec un halogénure de méthyle ou du sulfate de diméthyle.

8. Procédé selon la revendication 7, caractérisé en ce que l'on fait réagir la 3-méthylamino-4-amino-6-tert.-butyl-4,5-dihydro-1,2,4-triazine-5-one avec un halogénure de méthyle en présence d'un catalyseur à transfert de phase.

9. Procédé selon la revendication 8, caractérisé en ce que l'on fait réagir la 3-méthylamino-4-amino-6-tert.-butyl-4,5-dihydro-1,2,4-triazine-5-one avec un halogénure de méthyle en présence d'un solvant organique, d'une base forte et d'un catalyseur à transfert de phase.

10. Procédé selon la revendication 8, caractérisé en ce que l'on utilise en tant que catalyseur à transfert de phase un sel ou un hydroxyde d'ammonium quaternaire ou un sel de phosphonium.

11. Procédé selon la revendication 10, caractérisé en ce que l'on utilise en tant que catalyseur à transfert de phase un hydroxyde de benzyltrialkylammonium, un bisulfate de benzyltrialkylammonium, un halogénure de benzyltrialkylammonium, un hydroxyde de tétraalkylammonium, un bisulfate de tétraalkylammonium ou un halogénure de tétraalkylammonium.

12. Procédé selon la revendication 11, caractérisé en ce que l'on utilise en tant que catalyseur à transfert de phase un halogénure de tétraalkylammonium.

13. Procédé de préparation d'un produit pour la lutte contre les mauvaises herbes, caractérisé en ce que l'on mélange la 3,4-di-(méthylamino)-6-tert.-butyl-4,5-dihydro-1,2,4-triazine-5-one, composant actif, avec des véhicules et/ou d'autres additifs inertes.

14. Utilisation de la 3,4-di-(méthyalmino)-6-tert.-butyl-4,5-dihydro-1,2,4-triazine-5-one dans la lutte contre les croissances de végétaux indésirables.

15. Utilisation selon la revendication 14, dans la lutte contre les mauvaises herbes dans les cultures de végétaux utiles.

16. Utilisation selon la revendication 15, dans la lutte contre les mauvaises herbes dans les cultures de céréales, de maïs, de soja et de canne à sucre.

17. Utilisation selon la revendication 16, dans la lutte contre les mauvaises herbes dans les cultures de canne à sucre.

18. Utilisation selon la revendication 15, dans la lutte contre les mauvaises herbes monocotylédones.

19. Utilisation selon la revendication 15, dans la lutte contre les mauvaises herbes dicotylédones.

20. Procédé pour combattre les mauvaises herbes dans les cultures de végétaux utiles, caractérisé en ce que l'on applique sur les cultures de la 3,4-di-(méthylamino)-6-tert.-butyl-4,5-dihydro-1,2,4-triazine-5-one ou un produit contenant ce composé.

21. Procédé selon la revendication 20, caractérisé en ce que l'on applique la 3,4-di-(méthylamino)-6-tert.-butyl-4,5-dihydro-1,2,4-triazine-5-one ou un produit contenant ce composé en quantités correspondant à une quantité de 30 à 2 000 g de 3,4-di-(méthylamino)-6-tert.-butyl-4,5-dihydro-1,2,4-triazine-5-one par hectare.